Europäisches Patentamt

(19) European Patent Office

Office européen des brevets

(11) Publication number: **0 142 169 B1**

(12) **EUROPEAN PATENT SPECIFICATION**

(45) Date of publication of patent specification: **04.12.91**

(51) Int. Cl.5: **C12N 9/04**, C12P 7/60, //C12P19/02

(21) Application number: **84113750.8**

(22) Date of filing: **14.11.84**

The file contains technical information submitted after the application was filed and not included in this specification

(54) **2,5-Diketo-D-gluconic acid reductase.**

(30) Priority: **17.11.83 JP 217176/83**

(43) Date of publication of application:
**22.05.85 Bulletin 85/21**

(45) Publication of the grant of the patent:
**04.12.91 Bulletin 91/49**

(84) Designated Contracting States:
**BE CH DE FR IT LI NL**

(56) References cited:
**EP-A- 0 088 408**
**EP-A- 0 088 409**
**EP-A- 0 132 308**

**CHEMICAL ABSTRACTS, vol. 67, no. 23, 4th December 1967, page 9924, abstract no. 105462p, Columbus, Ohio, US; J. DE LEY: "5-Ketogluconic acid reductase", & METHODS ENZYMOL. 9, 200-3(1966)**

**AGRICULTURAL AND BIOLOGICAL CHEMISTRY, vol. 43, no. 1, January 1979, page 75; O. ADACHI et al.: "Crystallization and properties of 5-keto-D-gluconate reductase from Gluconobacter suboxydans"**

(73) Proprietor: **SHIONOGI & CO., LTD.**
**1-8, Doshomachi 3-chome Chuo-ku**
**Osaka 541(JP)**

(72) Inventor: **Sonoyama, Takayasu**
**2-13-13, Takeshirodai**
**Sakai-shi Osaka(JP)**
Inventor: **Kageyama, Bunji**
**3-12-4, Yamatedai**
**Ibaraki-shi Osaka(JP)**
Inventor: **Kobayashi, Kobee**
**31-7, 6-bancho Hiyoshidai**
**Takatsuki-shi Osaka(JP)**
Inventor: **Tanimoto, Masahiro 10-30-909, 3-chome**
**Motoyamaminami-cho Higashinada-ku**
**Kobe-shi Hyogo(JP)**

(74) Representative: **Vossius & Partner**
**Siebertstrasse 4 P.O. Box 86 07 67**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

SCIENCE, vol. 230, no. 4722, 11th October 1985, pages 144-149; S. ANDERSON et al.: "Production of 2-keto-L-gulonate, an intermediate in L-ascorbate synthesis, by a genetically modified Erwinia herbicola"

## Description

The present invention relates to purified 2,5-diketo-D-gluconic acid reductase (hereinafter, abbreviated as 25DKG-Rase) and its production.

The disclosed enzyme catalyzes in the presence of reduced nicotinamide adenine dinucleotide phosphate (abbreviated as NADPH) the reduction of 2,5-diketo-D-gluconic acid (abbreviated as 25DKG) to 2-keto-L-gulonic acid (abbreviated as 2KLG), the precursor of vitamin C. It also catalyzes the reduction of 5-keto-D-fructose (abbreviated as 5KF) to L-sorbose.

2KLG production from 25DKG by the use of cell extracts obtained from a microorganism which belongs to the genus Corynebacterium has been disclosed, for instance, in European Patent Publication No. 00 88 408 and UK Patent Publication No. 2 116 549. However, no disclosure has been made up to now on a purified enzyme having both the stated enzyme activities and commonly requiring NADPH.

Therefore, it is an object of the present invention to provide the purified 25DKG-Rase.

It is another object to provide a process for producing the purified 25DKG-Rase.

According to the present invention, there is provided a purified 25DKG-Rase having the following physico-chemical properties:

(a) enzyme activity: catalyzation in the presence of NADPH as a coenzyme,
(i) reduction of 25DKG or its salts to 2KLG or the corresponding salts thereof, and
(ii) reduction of 5KF to L-sorbose.
(b) specificities for substrates: specificities for 25DKG and 5KF.
(c) optimum pH: pH 6 -7
(d) pH stability: pH 5 -7
(e) molecular weight: 29, 000 ± 2, 000
(f) isoelectric point: pH 4.4 ± 03

In another embodiment of the present invention, there is provided a process for producing this purified enzyme by isolating it from disrupted cells of a microorganism which belongs to the genus Corynebacterium.

In the course of studies concerning enzymes which reduce 25DKG and which are carried by a number of 2KLG-producing microorganisms, the present inventors have found that a 25DKG-Rase obtained from 2KLG-producing strains which belong to the genus Corynebacterium has the catalytic activity, in the presence of NADPH, of (i)reducing 25DKG to 2KLG, and (ii)reducing 5KF to L-sorbose. Furthermore, they have completed the present invention by succeeding in isolating and purifying the novel enzyme which has undisclosed actions (compatible holding of both activities is a particularly unprecedented discovery).

Although the enzyme has the enzyme actions on 25DKG and 5KF, it does not reduce 5-keto-D-gluconic acid which shares the presence of keto-group at the 5 position that is common to these two substrates. In addition, it does not show reducing activity neither on ketose, such as D-fructose and L-sorbose having keto-group adjacent to the terminal alcoholic hydroxy group, nor on 2KLG or 2-keto-D-gluconic acid.

The enzyme is an intracellular enzyme extracted from the cells of the 2KLG producing strain belonging to the genus *Corynebacterium* . As the microorganisms useful in the process of the present invention, there is exemplified *Corynebacterium sp.* No.20A-77, ATCC 31,090, FERM-P 2,770, one of the 2KLG producing strains belonging to the genus *Corynebacterium*, and a mutant derived from this strain (for instance, a mutant defective in metabolizing 5-keto-D-gluconic acid, FERM-BP 108). In addition, *Corynebacterium sp.* No.T13, ATCC 31,089, *Corynebacterium sp.* No. K-106, ATCC 31,088, and any mutant derived from these strains may also be used as a source of the enzyme.

These microorganisms are deposited with the Fermentation Research Institute, Yatabe, Japan as FERM-Ps or FERM-BPs and/or with the American Type Culture Collection, Maryland, U.S.A. as ATCCs by the present inventors, and samples of these are available from those depositories under the provision of the Budapest Treaty.

Detailed Taxonomical descriptions of the strains No.20A-77, T-13 and K-106 are given, for instance, in United States Patent No. 3,959, 076 and the method of inductive mutation to FERM-BP 108 strain from the parent 20A-77 strain is disclosed in detail,for instance, in European Patent Publication No. 0088,408.

No particular restriction should be imposed on the composition of the nutrient medium used for the growth of the 2KLG-producing strains to produce 25DKG-Rase of the present invention. The medium should desirably contain carbon sources, nitrogen sources, other inorganic salts, and trace amounts of other factors which are assimilable by the strains. As the carbon source, sugars,such as D-glucose and sucrose, organic acid,such as gluconic acid, glycerol and the like may be used in an aqueous medium at 1 - 3 %. As the nitrogen source, polypeptone, corn steep liquor and the like may be used at 0.5 - 5 %. Phosphate, magnesium salt ,vitamin and trace amount of other metal salts essential for the growth may also be used.

3

The medium is inoculated with the previously described 2KLG-producing strain and this culture is incubated at 25-35°C for 15-30 hours for the propagation of the microoganisms.

In the following, one of the possible methods for extracting the intracellular enzyme from the microorganisms after the cultivation is described. Cells are first harvested from the broth by centrifugation and subsequently they are washed with water, physiological saline or a buffer solution of an appropriate pH. The washed cells are suspended in a buffer of an appropriate pH and are disrupted by means of sonication, a French press or treatment with an enzyme (lysozyme). A subsequent centrifugation will remove unbroken cells and cell debris from the solution and yields a supernatant (crude enzyme solution). Ammonium sulfate is added to the crude enzyme solution to salt out (30-70% saturated fraction).

The salted-out product is dissolved in a buffer appropriately prepared so that the enzyme may not be inactivated and the solution is subjected to dialysis to remove the remaining ammonium sulphate. Thereafter, the enzyme solution is subjected to (a) purification treatment(s), such as ion-exchange chromatography and affinity chromatography,to give the purified 25DKG-Rase, as a single component.

The following physico-chemical properties of the purified 25DKG-Rase were determined:

(1) Action:

$$25DKG + NADPH + H^+ ---\rangle 2KLG + NADP^+$$

$$5KF + NADPH + H^+ ---\rangle L\text{-sorbose} + NADP^+$$

As shown, NADPH is used as a hydrogen donor in the above reaction. Reduced nicotinamide adenine dinucleotide (NADH) may also be used as the hydrogen donor in the above reaction but the reaction velocity in that case is lowered to one to two hundred and fiftieth (1/250) or less in comparison with the reaction where NADPH is used.

(2) Specificities for substrates:

The enzyme shows specificities for 25DKG and 5KF. However, it does not reduce 5-keto-D-gluconic acid which shares a similarity with the two substrates and does not show any reducing action on ketoses having a keto-group adjacent to the terminal alcoholic hydroxy group such as D-fructose and L-sorbose and on compounds having a keto-group at the 2-position, such as 2KLG and 2-keto-D-gluconic acid.

No substantial production of 25DKG or 5KF from 2KLG or L-sorbose is recognized in the presence of NADP or NAD.

(3) Optimum pH: pH 6 - 7

(4) PH stability: pH 5 - 7

(determined on the basis of residual activities after treatments in 0.1 M dimethylglutaric acid buffer for pH 4-5, in 0.1 M Good buffer (PIPES) for pH 6-7 and in 0.1 M Tris-HCl buffer for ph 8-9 at 28°C for 30 minutes). Residual activity of 70 % or more is recognized in pH 5-7 while only those of 30 % or less are recognized in pH 4 or below ,and in pH 8 or above.

(5) Measurement of enzymatic activity:

The enzymatic activity is spectrophotometrically measured on the basis of the decrease in absorption at 340 nm which corresponds to the oxidation of NADPH as the result of the enzyme reaction at 30°C in 0.1 M Tris buffer (pH 7) containing 0.1 mM NADPH and 3.3 mM Ca-25DKG. One unit of the enzymatic activity is defined as the amount which oxidizes 1 μmole of NADPH for one minute.

(6) Range of reaction temperature; 25 - 45°C

(7) Inactivation conditions of pH and temperature:

96 % or more of the activity is lost after keeping the enzyme at pH 4 or below, or pH 8 or above and at 28°C for 2 hours.

(8) Inhibition, activation and stabilization:

4

The enzyme is inhibited by oxalate and scarcely inhibited by glyceroaldehyde. No remarkable increase in activation is observed by the addition of $Mg^{++}$ or $Mn^{++}$. It is stabilized by glycerol of a high concentration (30-50 %) or sucrose (1M).

(9) Molecular weight: 29,000 ± 2,000

(measured by SDS-polyacrylamide electrophoresis and gel filtration).

(10) Isoelectric point: pH 4.4 ± 0.3

(measured by isoelectric point electrophoresis).

(11) Km value: 2.2 ± 1.5 mM ( 25DKG substrate in 0.1 M Tris buffer (pH 7.0) at 30° C)

The 25DKG-Rase of the present invention is available for the uses where the above described enzyme action is demonstrated. 2KLG and L-sorbose can be produced from 25DKG and 5-keto-fructose,respectively, in the presence of the coenzyme, NADPH. In the case of 2KLG production, for instance, the reaction usually proceeds at 25 - 45° C and pH 6-7. The molar concentration of the substrate, 25DKG which participates in the reaction is equal to that of the hydrogen donor, NADPH and should be determined in accordance with the common knowledge relating to enzyme reaction. However, the molar concentration in the range of 20-200mM is considered to be appropriate.

The enzyme may preferably be allowed to react with the substrate in a concentration ranging from 0.5 to 5.0 unit/ml. In the reaction, the enzyme may also be used in an immobilized state with an appropriate carrier. Any means of immobilizing enzymes generally known to the art may be used. For instance, the enzyme may be bound directly to a membrane, granules or the like of a resin having (a) functional group(s), or it may be bound through bridging compounds having (a) bifunctional group(s), for example, glutaraldehyde, to the resin.

The time of said enzyme reaction should not particularly be limited, though 90 % or more of 25DKG of the substrate can be reduced to 2KLG in the reaction performed at 30 ° C for 60 -120 minutes.

Description of the Preferred Embodiments

By the examples given in the following, the present invention will be described in more detail.

Example 1

(1) Culture of cells

(i) Seed medium

A medium (60 ml) of the following composition was placed in a 500 ml flask and sterilized at 120° C for 20 minutes:

| | |
|---|---|
| D-glucose | 1.0 % |
| Yeast extract (Difco) | 0.5 % |
| Bacto-peptone (Difco) | 0.5 % |
| NaNO₃ | 0.1 % |
| KH₂PO₄ | 0.1 % |
| MgSO₄.7H₂O | 0.02 % |
| pH 7.0 | |

(ii) Medium for growth

A medium (20 L) of the following composition with 50 ppm of an antifoam (polypropylene glycol P-2000) was placed in a 30 L jar fermenter and sterilized at 120° C for 20 minutes:

| D-glucose | 2.0 % |
|---|---|
| Corn steep liquor | 3.0 % |
| Yeast extract | 0.1 % |
| NaNO$_3$ | 0.3 % |
| KH$_2$PO$_4$ | 0.06 % |
| pH 7.2 | |

(iii) Culture

Each of ten (10) seed media was inoculated with one loopful of the mutant (FERM-BP 108) derived from *Corynebacterium sp.* No.20A-77 and incubated in a rotary shaker (amplitude, 71 mm, 270 rpm.) at 28°C for 18 hours. The incubated media (10) were transferred to the medium for growth which was cultured at 28°C for 22 hours with an air flow rate of 0.5 vvm and agitation at 400 r.p.m. (OD = 19).

(2) Extraction of the Enzyme

The cells in the incubated broth obtained in (1) above were collected therefrom by centrifugation (Sharpless centrifuge; 10,000 g for 10 min.) and washed with 0.1 M tris buffer (hereinafter, abbreviated to as TB and being adjusted to pH 7.0 if not otherwise specified). The washed cells were suspended in 0.1 M TB so that the OD of the suspension was 150 and disrupted by means of sonication (160 watt/80 ml. for 7 min.). Unbroken cells and cell debris were removed from the sonicated solution by centrifugation (15,000 g for 30 min.) to give a supernatant of about 1 L (protein = 10.3 mg/ml, 25DKG-Rase = 0.63 units/ml).

(3) Purification of the Enzyme

(The following procedure was entirely performed at 4°C or below)

(i) Fractionation with ammonium sulfate

Ammonium sulfate was added to the supernatant obtained above (2) to 30 % saturation and the salted-out product was removed by centrifugation (10,000 g for 10 min.). To the thus obtained supernatant ammonium sulfate was added to give a 70 % saturation. After being cooled with ice for about 1 hour, the precipitated protein was collected by centrifugation (10,000 g for 10 min.). The protein was dissolved in 200 ml of 0.1 M TB and the solution was dialyzed against 0.02 M TB overnight.

(ii) Ion-exchanging chromatography

The diazlyzed solution (250 ml, 13.6 mg/ml protein) obtained in (i) above was applied to a DEAE-Sepharose CL-6B column (available from Pharmacia Finechemicals Co.) and subjected to chromatography under the following conditions:
Column size: 1.6x30 cm , Equilibrating liquid: 0.02 M TB, Eluent = 0.02 M TB(200 ml)------ 0.15 M NaCl/0.02 M TB (300 ml)------ 0.25 M NaCl/0.02 M TB(200 ml)
5 ml fractions of the eluate were collected and enzymatic activities of the respective fractions for substrates of Ca-25DKG and 5KF were measured for each fraction in a manner which will be described below in (5)-(ii). As a result, the enzymatic activities for both substrates were observed in 80 ml of eluate eluted with 0.25 M NaCl/0.02 M TB.
Then ammonium sulfate was added to the eluate to give a 70 % saturation to salt out the protein, the salted-out product was dissolved in 30 ml of 0.1 M TB and dialyzed against 0.02 M TB at 4°C overnight.

(iii) Affinity chromatography

The dialyzed solution obtained in (3)-(ii) above was applied to an Amicon Matrix Gel Red A column (available from Amicon Far East Limited) which had previously been equilibrated with 0.02 M TB and subjected to chromatography under the following conditions:
Column size: 1.6x19 cm, Washing liquid: 0.4 M NaCl/0.02 M TB(400 ml),Eluent = 0.5 M NaCl/0.02 M TB(450 ml) ----- 0.7 M NaCl/0.02 M TB(150 ml)----- 1 M NaCl/0.02 M TB(300 ml).

6

As a result of enzymatic activity measurements of the respective fractions (5 ml each) in the same manner as described in (3)-(ii) above, the enzymatic activities for both substrates, i.e. 25DKG and 5KF, were oberserved in 18 fractions eluted with 0.7 M-1.0 M NaCl/0.02 M TB. 225 ml of 0.02 M TB were added to the collected active fractions (90 ml) to lower the NaCl concentration in the solution and the mixture was applied again to an Amicon Matrix Gel Red A Column (1.9x13 cm). The column was washed with 120 ml of 0.2 M NaCl/0.02 M TB and eluted with 150 ml of 0.02 M TB containing 0.5 mM NADPH and 0.2 M NaCl.

The eluate was portioned to 5 ml fractions whose enzymatic activities were measured as described above. As a result, it was found that the fractions (about 35 ml) having high enzymatic activity were contained in those fractions (about 70 ml) which were eluted with 0.02 M TB containing 0.5 mM NADPH and 0.2 M NaCl.

### (iv) Concentration of enzyme and removal of NADPH

To the eluate obtained in (iii) above, ammonium sulfate was added to give a 30 % saturation and subsequently it was applied to a Phenyl Sepharose CL-4B column (0.9x3 cm, available from Pharmacia Finechemicals Co.) which had previously been equilibriated with 0.02 M TB containing ammonium sulfate in 30 % saturation. The column was washed with 0.02 M TB containing ammonium sulfate in 30 % saturation to completely remove NADPH. The removal was confirmed by the measurement of the absorption at 340 nm, the enzyme in the column was eluted with 0.02 M TB and 1 ml fractions were collected.

As a result of the enzymatic activity measurements of these fractions, high activity is observed within four (4) fractions. The four fractions collected were a purified enzyme solution and were used in the subsequent measurements. This solution showed 54 units/ml 25DKG-Rase activity and contained 0.58 mg/ml protein.

### (4) Purity of the purified enzyme solution

2-5 $\mu$l of the purified enzyme solution obtained in (3)above were analyzed by a SDS-polyacrylamide gel electrophoresis (separating gel; 10 % acrylamide, conditions of electrophoresis: 40 mA at room temperature for 1 hr.) wherein the enzyme yielded a single band at a position which corresponds to a molecular weight of 29,000±2,000.

As a result of further gel filtration analyses (Sephadex G-100), an enzymatic activity was observed in the fractions which correspond to a molecular weight of 29,000±2,000.

5 $\mu$l of the enzyme solution were then subjected to isoelectric point electrophoresis (polyacrylamide gel; conditions of electrophoresis (a); pH interval 2.5 - 5; anode electrolyte = 0.1 M $H_2SO_4$;cathode electrolyte = 0.1 M NaOH, 6W, 2.700 VH: conditions of electrophoresis (b): pH interval 4.0-6,5; anode electrolyte = 0,04 M DL-glutamic acid, cathode electrolyte = 0.2 M NaOH, 25W, 2,600 VH; temperature, 18 - 20° C) with the result that the enzyme migrated in a single band which corresponds to pH 4.4±0.2. From the previously described data it was confirmed that the purified enzyme solution solely contains 25DKG-Rase.

### (5) Measurements of Physico-chemical properties

### (i) Enzyme reaction

The purified enzyme solution (10 $\mu$l) obtained above (3) was mixed with 25 $\mu$ moles of a substrate ( Ca-25DKG or 5KF ) and 3.0 ml of 0.01 M TB (pH 7) containing 15 $\mu$ moles of NADPH and the mixture was allowed to react at 30° C for 60 minutes. After the reaction, the reaction mixture was analyzed by paper chromatography (developing solvent; phenol:formic acid:water = 75:4:25, chromogenic agent: AHF solution, prepared by dissolving 0.93 g of anilin and 1.66 g of phthalic acid in 100 ml of water-saturated n-butanol and heating at 105° C for 2 minutes) and by gas chromatography ( column; SE-52, carrier gas: Helium, sample: trimethylsilylated derivative). As a result of the analyes, in both chromatographies it was found that only 2KLG is produced from 25DKG and only L-sorbose is produced from 5KF. In Table 1, the results of the quantitative determination of the products by gas chromatography are shown.

7

Table 1

| Reduction of 25DKG and 5KF by purified enzyme solution | | |
|---|---|---|
| Substrate | Product | Concentration of the product |
| Ca-25DKG<br>5KF | Ca-2KLG<br>L-sorbose | 845 $\mu$ g/ml<br>795 $\mu$ g/ml |

(ii) Substrate specificity

2.9 ml of 0.1 M TB (pH 7) containing 0.3 $\mu$ moles of NADPH were placed in a quartz cuvette having 1 cm optical length and 1 - 5 $\mu$l of the enzyme solution obtained as described in (3) above were added. To the resulting mixture 0.1 ml of each of the various substrate solutions (0.1 M) were added while keeping the reaction mixture at 30°C. Reactivities of the enzyme on the respective substrates were determined by successive measurement or the decrease in absorption at 340 nm, on the basis of the decreases for 1 minute. The results of the measurement are shown in the following Table 2:

## Table 2

### Specificity of 25DKG-Rase for various substrates

| Substrate | Activity of the purified enzyme solution ( 1 ml ) | |
|---|---|---|
| Ca-25DKG | 54 | unites |
| 5KF | 94 | " |
| K-5-keto-gluconate | 0 | " |
| D-fructose | 0 | " |
| L-sorbose | 0 | " |
| Ca-2-keto-D-gluconate | 0 | " |
| Na-2KLG | 0 | " |

It is evident from the above table that the enzyme of the present invention shows a high activity for 25DKG or 5KF but does not show any reductive activity for 5-keto-D-gluconic acid, D-fructose, L-sorbose, 2-keto-D-gluconic acid and 2KLG.

(iii) Coenzyme (hydrogen donor)

NADH was substituted for the NADPH in the reaction system of (5)-(ii) and the enzymatic activities of the 25DKG-Rase for the substrates Ca-25DKG and 5KF were measured. The results of the measurements are shown in Table 3.

## Table 3

### The effect of coenzymes on 25DKG-Rase activity

| Substrate | Enzymatic activity (units/1 ml of the purified enzyme solution) | |
| --- | --- | --- |
| Coenzyme: | NADPH | NADH |
| Ca-25DKG | 54 | 0.2 ) |
| 5KF | 94 | 0.2 ) |

As shown in the above table, the enzyme of the present invention showed enzymatic activity of 54 units/1 ml of the purified enzyme solution on Ca-25DKG when the enzyme is used together with NADPH,whereas it showed enzymatic activity of only 0.2 unit or less if combined with NADH. Likewise, the activity of 94 units on 5KF with NADPH decreased to 0.2 unit or less with NADH.

(iv) Optimum pH

In order to clarify the correlation between the reaction rate of the 25DKG-Rase and pH, the enzymatic activities were measured by using the following buffers instead of the buffer of the reaction system in (5)-(ii).
pH 4.0 - 5.0: 0.1 M 3,3-dimethylglutaric acid buffer.
pH 6.0 - 7.0: 0.1 M Good buffer (PIPES: piperazine-N,N-bis-(2-ethane) sulfonic acid)
pH 7.0 - 9.0: 0.1 M Tris buffer.
The results of the measurements of the enzymatic activities on the substrates Ca-25DKG and 5KF at the respective pHs are shown in Table 4 below.

## Table 4

### 25DKG-Rase activity and pH in the enzyme reaction

| pH | Buffer | Enzymatic activity[a] (units/ml) | |
|---|---|---|---|
| | | Substrate: Ca-25DKG | 5KF |
| 4.0 | 3,3-dimethyl-glutaric acid | 1 | 2 |
| 5.0 | " | 22 | 33 |
| 6.0 | Good (PIPES) | 59 | 96 |
| 6.5 | " | 57 | 95 |
| 7.0 | " | 53 | 92 |
| " | Tris | 54 | 93 |
| 7.5 | " | 44 | 68 |
| 8.0 | " | 32 | 48 |
| 8.5 | " | 11 | 27 |
| 9.0 | " | 7 | 13 |

(a) Enzymatic activity of units per 1 ml of the purified enzyme solution.

As evident from the above table, the enzyme showed the highest enzymatic activity at pH 6 - 7.

(v) Temperature dependence of the activity

The reaction rates in the reaction system of (5)-(ii) using Ca-25DKG as the substrate were measured at temperatures varying from 15 to 50°C. The results thereof are shown in Table 5 below:

## Table 5

### 25DKG-Rase activity and temperature

| Temperature °C | Enzymatic activity(units/ml) |
| --- | --- |
| 15 | 14 |
| 25 | 29 |
| 27.5 | 44 |
| 30 | 54 |
| 32.5 | 59 |
| 35 | 64 |
| 40 | 67 |
| 45 | 44 |
| 50 | 10 |

Enzymatic activity: that per 1 ml of the purified enzyme solution on the substrate Ca-25DKG at pH 7.

As evident from the above table the activity of the enzyme of the present invention increases with increasing temperature up to 40°C and decreases with a further increase of the temperature to 45°C or more.

(vi) Measurement of the Km value

In the procedure described in (5)-(ii) above, the velocities of reducing reactions with varying concentrations of Ca-25DKG from 0.01 M to 0.25 M, respectively, were measured to determine the Km value for 25DKG. As a result the Km value was found to be 1.8 ± 1.0 mM.

Example 2

(1) Culture of cells

(i) Medium for growth

A medium (12 L) of the following composition is placed in a 20 L jar fermenter and sterilized at 120°C for 20 minutes:

EP 0 142 169 B1

| D-glucose | 1.0 % |
| Corn steep Liquor | 1.0 % |
| Yeast extract (Difco) | 0.2 % |
| Polypeptone (Difco) | 0.5 % |
| $KH_2PO_4$ | 0.1 % |
| $MgSO_4.7H_2O$ | 0.02 % |
| pH 7 | |

(ii) Culture

Two seed cultures for each of *Corynebacterium sp.* No.K-106 (ATCC 31,088, abbreviated as K-106) and *Corynebacterium sp.* No. T-13 (ATCC 31,089, abbreviated as T-13) were prepared with the medium and according to the procedures described in Example 1 (1).

Each of these seed cultures was inoculated in each of the media prepared in (i) above and cultured at 28°C for 18 hours with an aeration of 0.5 vvm and agitation at 400 r.p.m. The amount of cells given as OD at the end of the culture was as follows: OD = 11.8 for Strain No. K-106; OD = 12.2 for No.T-13.

(2) Extraction of enzyme

Cell suspensions of OD = 150 in 0.02 M TB of Strains- Nos.K-106 and T-13 were prepared by washing the separated cells from the cultured brothes in accordance with the method described in Example 1-(2). The cells in the suspensions were disrupted using a French press (700 kg/cm²). The solutions of disrupted cells were subjected to centrifugation (15,000 g, 30 min.) as described in Example 1-(2) to remove unbroken cells and cell debris. As a result, cell extracts having the following enzymatic activities were obtained.

| Strain | Amount of extract | Protein | 25DKG-Rase activity |
|--------|-------------------|---------|---------------------|
| K-106 | 400 ml | 10 mg/ml | 2.1 units/ml |
| T-13 | 400 ml | 12 mg/ml | 2.0 units/ml |

(3) Purification of enzyme

(i) Salting-out with ammonium sulfate

Proteins corresponding to the fractions salted out with ammonium sulfate of 30 - 70 % saturation were collected as described in Example 1- (3) and were dissolved in 0.02 M TB. The resulting solutions were diazlyzed against 0.02 M TB at 4°C overnight.

As a result, dialyzed solutions having the following enzymatic activities were obtained:

| Strain | Amount of enzyme solution | Protein | 25DKG-Rase activity |
|--------|---------------------------|---------|---------------------|
| K-106 | 74 ml | (Not tested) | 7.0 units/ml |
| T-13 | 88 ml | 11.1 mg/ml | 4.8 units/ml |

(ii) Ion-exchanging chromatography

The enzyme solutions were treated as described in Example 1-(3) -(ii). As a result, eluates having the following enzymatic activities were obtained:

12

| Strain | Amount of enzyme solution | Protein | 25DKG-Rase activity |
|--------|---------------------------|---------|---------------------|
| K-106 | 49 ml | 0.60 mg/ml | 2.4 units/ml |
| T-13 | 52 ml | 0.94 mg/ml | 4.1 units/ml |

(iii) Affinity chromatography

Ammonium sulfate was added to obtain a 30% saturation to each of the eluates obtained in (ii) above, and the enzymes therein were concentrated and desalted by Phenyl Sepharose gel column chromatography in accordance with the method described in Example 1-(3)-(iv). The concentrated enzyme solutions were purified twice by affinity chromatography with Amicon Matrix Gel Red A Column, as described in Example 1-(3)-(iii). As a result, the 25DKG-Rases derived from both strains were eluted in the fractions collected with the eluant of 0.7-1 M NaCl concentration in the first chromatography and in the fractions collected with 0.5 mM NADPH in the second chromatography.

The eluates were desalted and concentrated after the NADPH removal by the method described in Example 1-(3)-(iv). As a result , the purified enzyme solutions having the following activities were obtained:

| Strain | Amount of enzyme solution | Protein | 25DKG-Rase activity |
|--------|---------------------------|---------|---------------------|
| K-106 | Ca 1 ml | 0.25 mg/ml | 23.8 units/ml |
| T-13 | Ca 2 ml | 0.17 mg/ml | 15.1 units/ml |

The obtained enzyme solutions (2-10 $\mu$ 1) were assayed by means of SDS-polyacrylamide gel electrophoresis and isoelectric electrophoresis as described in Example 1-(4). Each of the enzymes showed a single band. The purified enzyme solutions were subjected to the following determination of the properties.

(4) Physico-chemical properties of the enzymes

The measurements of the physico-chemical properties were conducted as described in Example 1-(4)--(5).

(i) Molecular weight

By SDS-polyacrylamide gel electrophoresis, the molecular weights of both the 25DKG-Rases derived from Strain K-106 and T-13 were found to be 29,000 ± 2,000.

(ii) Isoelectric point

The results of the isoelectric point electrophoreses for the enzymes derived from Strains K-106 and T-13 were pH 4.4 ± 0.2 and pH 4.5 ± 0.2, respectively.

(iii) Substrate specificity

Each of the enzymes produced only Ca-2KLG from Ca-25DKG and only L-sorbose from 5KF. Nothing was produced from 5-keto-D-gluconic acid, D-fructose, L-sorbose, 2KLG and 2-keto-D-gluconic acid.

(iv) Enzymatic activity

The enzyme from Strain K-106 had an activity for Ca-25DKG of 23.8 units/ml (95 units/ mg protein) and that for 5KF of 38.3 units/ml (152 units/mg protein). The enzyme from Strain T-13, however, had an activity for Ca-25DKG of 15.1 (89 units/ mg protein) and that for 5KF of 39 units/ml (229 units/mg protein).

(v) Coenzyme (hydrogen donor)

The results of the measurements of the enzymatic activity of both enzymes for Ca-25DKG using NADH as the coenzyme resulted in an activity of 0.1 unit/ml or less in either case.

(vi) Optimum pH

Enzymatic activities of 25DKG-Rases obtained from K-106 and T-13 were measured at varying pH from 4 to 9 (substrate: Ca-25DKG, reaction temperature: 30° C). The results thereof are shown in Table 6 below:

## Table 6

### Enzymatic activities of 25DKG-Rases obtained from K-106 and T-13 and pH of the enzyme reaction

| pH | Enzymatic activity (relative activity)* | |
|-----|-------|-------|
| | K-106 | T-13 |
| 4.0 | 1 | 1 |
| 5.0 | 48 | 65 |
| 6.0 | 103 | 104 |
| 7.0 | 100 | 100 |
| 8.0 | 70 | 64 |
| 9.0 | 6 | 19 |

\* Activity at each pH defined on the basis that the activity is 100 at pH 7, $30^{\circ}$C.

From the above table it can be taken that the optimum pH of each of the 25DKG-Rases obtained from both strains is pH 6 - 7.

(vii) Temperature dependence of the activity

The enzymatic activities of 25DKG-Rases obtained from K-106 and T-13 were measured at varying temperature (pH 7.0, substrate:Ca-25DKG). The results are shown in Table 7 below.

## Table 7

### Enzymatic activity of 25DKG-Rases obtained from
### K-106 and T-13 and reaction temperature

| Temperature | Enzymatic activity (relative activity*) | |
| --- | --- | --- |
| (°C) | K-106 | T-13 |
| 22 | 46 | 64 |
| 25 | 69 | 79 |
| 28 | 86 | 92 |
| 30 | 100 | 100 |
| 35 | 115 | 115 |
| 40 | 119 | 133 |
| 45 | 93 | 128 |
| 50 | 44 | 92 |

**\* As defined in the former Table 6.**

From the data given in table 7 it is evident that the activities of 25DKG-Rases obtained from both strains in each case increase with increasing temperature up to 40°Cand decrease with an increase of the temperature to more than 45°C.

(viii) Km value

Km values of both enzymes on 25DKG measured by using purified enzyme solutions of K-106 and T-13 were 1.7 ± 1.0mM and 2.6 ± 1.0 mM,respectively.

**Claims**

1. Purified 2,5-Diketo-D-gluconic acid reductase having the following physico-chemical properties:
   (a)enzyme activity: catalyzation, in the presence of NADPH as a coenzyme,
      (i)reduction of 2, 5-diketo-D-gluconic acid or its salts to 2-keto-L-gulonic acid or the corresponding salts thereof, and
      (ii)reduction of 5-keto-D-fructose to L-sorbose.
   (b)specificities for substrates: 2, 5-diketo-D-gluconic acid and 5-keto-D-fructose.
   (c)optimum pH: pH 6 -7
   (d)pH stability: pH 5 -7
   (e)molecular weight: 29, 000 ± 2, 000
   (f)isoelectric point: pH 4.4 ± 0.3

2. A process for the production of the 2,5-diketo-D-gluconic acid reductase according to claim 1 which comprises culturing a microorganism belonging to the genus *Corynebacterium* in a nutrient medium containing carbon and nitrogen sources and inorganic salt, and then isolating the thus produced 2,5-diketo-D-gluconic acid reductase from disrupted cells of the microorganism.

**Revendications**

1. Réductase d'acide 2,5-dicéto-D'gluconique purifiée ayant les propriétés physico-chimiques suivantes :
    (a) activité enzymatique : catalysée, en présence de NADPH comme coenzyme,
        (i) réduction d'acide 2,5-dicéto-D-gluconique ou de ses sels en acide 2-céto-L-gulonique ou ses sels correspondants, et
        (ii) réduction de 5-céto-D-fructose en L-sorbose
    (b) spécificités pour substrats : acide 2,5-dicéto-D-gluconique et 5-céto-D-fructose
    (c) pH optimal : pH 6-7
    (d) stabilité au pH : pH 5-7
    (e) poids moléculaire : 29.000 ± 2.000
    (f) point isoélectrique : pH 4,4 ± 0,3.

2. Procédé de production de la réductase d'acide 2,5-dicéto-D-gluconique suivant la revendication 1, qui comprend la culture d'un micro-organisme appartenant au genre Corynebacterium dans un milieu nutritif contenant des sources de carbone et d'azote et du sel inorganique, et ensuite l'isolement de la réductase d'acide 2,5-dicéto-D-gluconique ainsi produite des cellules rompues du microorganisme.

**Patentansprüche**

1. Gereinigte 2,5-Diketo-D-gluconsäure-Reduktase mit den folgenden physikalisch-chemischen Eigenschaften:
    (a) Enzymaktivität: Katalyse in Gegenwart von NADPH als Coenzym,
        (i) Reduktion der 2,5-Diketo-D-gluconsäure oder ihrer Salze zu 2-Keto-L-gulonsäure oder den entsprechenden Salzen davon, und
        (ii) Reduktion von 5-Keto-D-fructose zu L-Sorbose;
    (b) Spezifität für Substrate: 2,5-Diketo-D-gluconsäure und 5-Keto-D-fructose;
    (c) pH-Optimum: pH 6-7;
    (d) pH-Stabilität: pH 5-7;
    (e) Molekulargewicht: 29 000 ± 2000;
    (f) isoelektrischer Punkt: pH 4,4 ± 0,3.

2. Verfahren zur Herstellung der 2,5-Diketo-D-gluconsäure-Reduktase gemäß Anspruch 1, umfassend die Züchtung eines Mikroorganismus, der zur Gattung Corynebacterium gehört, in einem Nährmedium, das Kohlenstoff- und Stickstoffquellen und anorganisches Salz enthält, und Isolierung der so erhaltenen 2,5-Diketo-D-gluconsäure-Reduktase aus den aufgebrochenen Zellen des Mikroorganismus.